# EUROPEAN PATENT SPECIFICATION

(11) **EP 3 562 307 B1**
(45) Date of publication and mention of the grant of the patent: **30.12.2020**
(21) Application number: 17823027.2
(22) Date of filing: 23.11.2017
(51) Int. Cl.: A01N 41/04, A01N 59/16, A01N 37/02, A01N 25/30, A01P 1/00, A61L 2/16, A61L 2/18

(54) **AN ANTIMICROBIAL COMPOSITION COMPRISING A SILVER COMPOUND**
ANTIMIKROBIELLE ZUSAMMENSETZUNG ENTHALTEND EINE SILBERVERBINDUNG
COMPOSITION ANTIMICROBIENNE COMPRENANT UN COMPOSE D'ARGENT

(30) Priority: 27.12.2016 EP 16206932
(43) Date of publication of application: 06.11.2019
(73) Proprietor: Unilever N.V., 3013 AL Rotterdam (NL); Unilever PLC, London, Greater London EC4Y 0DY (GB)
(72) Inventor: APPAVOO, Shanthi, Bangalore 560066 (IN); IYER, Vidula, Bangalore 560066 (IN); SALGAONKAR, Neha, Bangalore 560066 (IN)
(74) Representative: van den Brom, Coenraad Richard
(86) International application number: PCT/EP2017/080160
(87) International publication number: WO 2018/121933

(56) References cited:
- WO-A1-2014/170187
- WO-A1-2015/113782
- WO-A1-2016/050493

## Description

### Technical Field

The present invention relates to an antimicrobial composition and more particularly an antimicrobial composition for cleansing applications that provides antimicrobial efficacy in relatively short contact times.

### Background of the invention

Antimicrobial benefits of soap based cleaning compositions associated with the removal of organisms from a surface through the cleansing/detergency action of such products. In most of the cases for obtaining adequate and effective antimicrobial efficacy the contact/cleansing time needs to be sufficiently longer. However, the consumer's habit of washings hands/body parts or any other surface is not for prolonged time. Furthermore, the biocidal action of soap compositions against gram-positive bacteria is considerably more limited within the contact times typical of product use, generally under 1 minute, and more commonly of the order of 30 seconds or less.

Various routes for improving antimicrobial activity of soap based cleaning compositions known in the art:
WO 2010/046238 (Unilever, 2010) discloses an antimicrobial composition for cleansing or personal care. It is an object of the present invention to provide antimicrobial compositions that have relatively fast antimicrobial action. Present inventors have surprisingly found that compositions comprising selected ingredients, namely thymol and terpineol, in selective propositions provide relatively quick antimicrobial action.
US 2004/0014818 (Boeck, 2004) discloses a bactericidal preparation in the form of a solution, cream or ointment compounded from photosynthesized hydrocarbons, isolates from hydrocarbons, 2-hydroxy-1-isopropyl-4-methyl benzene (thymol) and butylated hydroxytoluene and exemplifies many compositions, each having from 10 to 20 compounds having anti-bacterial efficacy.
US2008014247A (Lu et al., 2008) discloses a composition having metal containing material, stearic acid and a pharmaceutically acceptable carrier to treat conditions caused by gram-positive, gram-negative, fungal pathogens and/or antibiotic-resistant bacteria. It further provides a method for inhibiting biofilm proliferation. The metal containing material can be silver.
US3050467 B1 (Horowitz et al. 1962) discloses an antimicrobial cleansing composition consisting essentially of a mixture of a water-soluble soap and a silver salt of partially depolymerized alginic acid. The composition provides synergestic antimicrobial activity.
WO 2015/113785 (Unilever, 2015) discloses a cleansing composition having pH of at least 9, said composition comprising: (i) 20 to 85 wt. percent anionic surfactant; and, (ii) a silver (I) compound having silver ion solubility (in water at 25 degrees C) of at least 1 x 10-4 mol/L, at a level equivalent to silver content of 0.01 to 100 ppm, wherein the free alkali content of said composition is less than 0.01 percent. The composition is a robust and improved cleansing composition with a stable colour.
WO 2014/170187 (Unilever, 2014) discloses a soap bar comprising: (a) 25 to 85% by weight, based on the total weight of the bar, of fatty acid soap; (b) 0.1 to 100 ppm by weight, based on the total weight of the bar, of at least one silver (I) compound having a selected silver ion solubility, wherein at 25°C, a 1 wt% solution of the bar in water has a pH of from 9 to 11.

Further antimicrobial compositions are disclosed in WO 2015/113782 and WO 2016/050493. Use of relatively high amount of silver compounds tends to affect the aesthetic properties of the formulation. Silver compounds are also considered to be not environmental-friendly, hence reduced amount of its uses is desirable.

Thus an object of the present invention is to provide an antimicrobial cleansing composition that provides biocidal activity in relatively short contact times of 1 minute to 10 seconds.

Another object of the present invention is to provide an antimicrobial cleansing composition which provides antimicrobial activity at very low concentration of silver compound.

A further object of the present invention is to provide an antimicrobial cleansing composition, which has consumer-acceptable aesthetic properties.

The present inventors while working extensively on this have surprisingly found that a composition comprising a particular amount of selected silver compounds, a salt of a sulphonic acid and a salt of fatty acid provides a synergistic antimicrobial composition with good antimicrobial efficacy in shorter contact time thereby satisfying one or more of the above said objects.

### Summary of the invention

In a first aspect, the present invention provides an antimicrobial composition comprising:
a) 0.1 to 100 ppm by weight of a silver compound;
b) a salt of a sulphonic acid ; and,
c) 1 to 85% by weight of a salt of fatty acid,
wherein, the sulphonic acid is an aromatic sulphonic acid, wherein the salt of the sulphonic acid is selected from sodium toluene sulphonates, sodium xylene sulphonates, napthalene sulphonates or mixtures thereof.

In a second aspect, the present invention provides a method of cleaning or disinfecting a surface comprising the steps of applying a composition of the first aspect on to said surface and at least partially removing the composition from the surface.

Any feature of one aspect of the present invention may be utilized in any other aspect of the invention. The word "comprising" is intended to mean "including" but not necessarily "consisting of" or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed description of the invention

The present invention provides an antimicrobial composition comprising:
a) 0.1 to 100 ppm by weight of a silver compound;
b) a salt of a sulphonic acid ; and,
c) 1 to 85% by weight of a salt of fatty acid
wherein, the sulphonic acid is an aromatic sulphonic acid, wherein the salt of the sulphonic acid is selected from sodium toluene sulphonates, sodium xylene sulphonates, napthalene sulphonates or mixtures thereof.

Antimicrobial composition as mentioned herein above preferably means any composition which is capable of killing or at least cause substantial reduction of the common disease-causing microbes. The common disease-causing gram-positive organisms includes *Staphylococcus, Streptococcus* and *Enterococcus* spp. Some of common disease-causing gram-negative organisms includes *Escherichia coli, Salmonella, Klebsiella* and *Shigella. Escherichia coli* and *Salmonella* can cause severe gastrointestinal illnesses.

### Silver compound:

The present invention employs at least one silver compound. The silver compound may preferably be selected from silver (I) compounds. The antimicrobial cleansing composition preferably includes 0.1 to 100 ppm, more preferably 0.5 to 50 ppm and most preferably 0.5 to 10 ppm silver compounds. The amount of silver compound as mentioned above is by weight of total silver compound.

The silver compounds are preferably water-soluble wherein the silver ion solubility at least 1.0 x10⁻⁴ mol/L (in water at 25°C). Silver ion solubility, as referred to herein, is a value derived from a solubility product (Ksp) in water at 25°C, a well known parameter that is reported in numerous sources. More particularly, silver ion solubility [Ag+], a value given in mol/L may be calculated using the formula: $\left[Ag+\right] = {\left(Ksp\cdot x\right)}^{\left(1/\left(x+1\right)\right)}$ wherein Ksp is the solubility product of the compound of interest in water at 25°C, and x represents the number of moles of silver ion per mole of compound. It has been found that silver (I) compounds having a silver ion solubility of at least 1 x 10⁻⁴ mol/L in are preferable for use herein. Silver ion solubility values for a variety of silver compounds are given in Table 1:

**TABLE 1**

| Silver Compound | X | Ksp (mol/L in water at 25 °C) | Silver Ion Solubility [Ag+] (mol/L in water at 25 °C). |
|---|---|---|---|
| Silver nitrate | 1 | 51.6 | 7.2 |
| Silver acetate | 1 | 2.0 x 10⁻³ | 4.5 x 10⁻² |
| Silver sulfate | 2 | 1.4 x 10⁻⁵ | 3.0 x 10⁻² |
| Silver benzoate | 1 | 2.5 x 10⁻⁵ | 5.0 x 10⁻³ |
| Silver salicylate | 1 | 1.5 x 10⁻⁵ | 3.9 x 10⁻³ |
| Silver carbonate | 2 | 8.5 x 10⁻¹² | 2.6 x 10⁻⁴ |
| Silver citrate | 3 | 2.5 x 10⁻¹⁶ | 1.7 x 10⁻⁴ |
| Silver oxide | 1 | 2.1 x 10⁻⁸ | 1.4 x 10⁻⁴ |
| Silver phosphate | 3 | 8.9 x 10⁻¹⁷ | 1.3x 10⁻⁴ |
| Silver chloride | 1 | 1.8 x 10⁻¹⁰ | 1.3 x 10⁻⁵ |
| Silver bromide | 1 | 5.3 x 10⁻¹³ | 7.3 x 10⁻⁷ |
| Silver iodide | 1 | 8.3 x 10⁻¹⁷ | 9.1 x 10⁻⁹ |
| Silver sulfide | 2 | 8.0 x 10⁻⁵¹ | 2.5 x 10⁻¹⁷ |

A preferred silver(I) compound is selected from silver oxide, silver nitrate, silver acetate, silver sulfate, silver benzoate, silver salicylate, silver carbonate, silver citrate and silver phosphate, more preferably the silver compound is silver oxide, silver sulfate or silver citrate and still further preferred silver(I) compound is silver oxide or silver sulphate.

The preferred silver compound may be selected from group consisting of silver oxide, silver nitrate, silver acetate, silver sulfate, silver benzoate, silver salicylate, silver carbonate, silver citrate and silver phosphate

The silver compound also may preferably be a complex of silver. The silver complex may be formed by reacting silver with one or more of a chelating agent. Chelates are characterized by coordinate covalent bonds. These occur when unbonded pairs of electrons on non- metal atoms like nitrogen and oxygen fill vacant d-orbitals in the metal atom being chelated. Valence positive charges on the metal atom can be balanced by the negative charges of combining amino acid ligands. The bonding of an electron pair into vacant orbitals of the metal allows for more covalent bonding than the valence (or oxidation number) of the metal would indicate. Forming bonds this way is called coordination chemistry. This allows chelates to form, providing that the ligands can bond with two or more moieties within the same molecule and providing that proper chemistry promoting chelation is present. An important factor is the strength of the complex formed between the metal ion and the chelating agent. This determines whether the complex will be formed in the presence of competing anions. The stability or equilibrium constant (K), expressed as log K, has been determined for many metals and chelating agents. The higher the log K values, the more tightly the metal ion will be bound to the chelating agent and the more likely that the complex will be formed.

Preferred chelating agents are ethylene diamine tetraacetic acid (EDT A), ethylene diamine dissuccinate (EDDS), N, N-bis (carboxymethyl) glutamic acid (GLDA), Diethylenetriaminepentaacetic acid (DTPA), Nitrilotriacetic acid (NTA) and Ethanoldiglycinic acid ((EDG). DTPA is particularly preferred and especially in combination with Silver. Chelating agents are usually used in the form of their salts with a metal. For example, EDTA is used in the form of disodium or tetrasodium salt. Accordingly, it is preferred to use a salt form of a chelating agent over the natural acid form. Preferably, the molar ratio of silver to the chelating agent is 1:0.25 to 1:10, more preferably 1: 0.5 to 1:5 and most preferably 1:1 to 1:3.

The amount of silver as mentioned is irrespective of its oxidation state.

Preferably, in the disclosed antimicrobial cleansing composition silver compound is present at levels not less than 0.4 ppm, still preferably not less than 0.5 ppm and further preferably not less than 1 ppm and it is preferred that the silver compound in the composition is present at levels not more than 80 ppm, more preferably not more than 50ppm, further preferably not more than 20 ppm and still further preferably not more than 10 ppm and most preferably not more than 5 ppm. It is highly preferred that the silver compound in the antimicrobial cleansing composition is present at 0.5 to 5 ppm.

### Salt of a sulphonic acid:

The composition of the present invention comprises a salt of a sulphonic acid. It is a member of organosulphur compound. The general structure of a sulphonic acid is as follows: 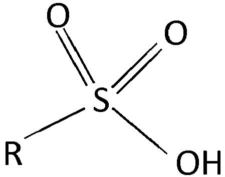 Wherein R can preferably be alkyl or aryl group. The salts of a sulphonic acid is known as sulphonates.

For the purpose of the present invention the sulphonic acid is selected from an aromatic sulphonic acid. The salt of a sulphonic acid is selected from sodium toluene sulphonates, sodium cumene sulphonates, sodium xylene sulphonates, naphthalene sulphonates or mixtures thereof. Also disclosed, the salt of a sulphonic acid may also be a silver salt of a sulphonic acid. This is not part of the invention as the present inventors intend to lower the amount of silver used in a personal cleansing formulation. When the silver salt of a sulphonic acid is used, the amount of silver is in addition to the amount that is mentioned in the previous section for a silver compounds.

The amount of salt of a sulphonic acid preferably is in the range of 0.1 to 20%, more preferably 0.1 to 15%, further more preferably 0.1 to 10% and most preferably 1 to 8% by weight of the composition.

### Salt of Fatty acid:

The composition of the present invention also comprises a salt of fatty acid. A salt of fatty acid is nothing but soap. It may also be called as fatty acid soap. The term "fatty acid soap" or, more simply, "soap" is used here in its popular sense, i.e., salts of aliphatic alkane- or alkene monocarboxylic fatty acids preferably having 6 to 22 carbon atoms, and more preferably 8 to 18 carbon atoms.

Usually a blend of fatty acids is used to get a blend of fatty acid soaps. The term "soap" refers to sodium, potassium, magnesium, mono-, di- and tri-ethanol ammonium cation or combinations thereof. In general, sodium soaps are preferred in the compositions of this invention, but up to 15% or even more of the soap content may be some other soap forms such as potassium, magnesium or triethanolamine soaps.

Preferably, the fatty acid blend is made from fatty acids that may be different fatty acids, typically fatty acids containing fatty acid moieties with chain lengths of from C8 to C22. The fatty acid blend may also contain relatively pure amounts of one or more fatty acids. Suitable fatty acids include, but are not limited to, butyric, caproic, caprylic, capric, lauric, myristic, myristelaidic, pentadecanoic, palmitic, palmitoleic, margaric, heptadecenoic, stearic, oleic, linoleic, linolenic, arachidic, gadoleic, behenic and lignoceric acids and their isomers.

The fatty acid blend preferably includes relatively high amounts (e.g., at least 3%, preferably at least 10%) of capric and lauric acids. Further preferably the fatty acid blend includes low levels of myristic acid, (e.g. preferably less than 4% by wt.) which generally provides good lathering property.

In preferred embodiments, the fatty acid blend has proportion of capric acid to lauric acid ranging from 0.5 to 1 to 1.5 to 1.

Soaps having the fatty acid distribution of coconut oil and palm kernel oil may provide the lower end of the broad molecular weight range. Those soaps having the fatty acid distribution of peanut or rapeseed oil, or their hydrogenated derivatives, may provide the upper end of the broad molecular weight range.

It is preferred to use soaps having the fatty acid distribution of coconut oil or tallow, or mixtures thereof, since these are among the more readily available triglyceride fats. The proportion of fatty acids having at least 12 carbon atoms in coconut oil soap is about 85 %. This proportion will be greater when mixtures of coconut oil and fats such as tallow, palm oil, or non-tropical nut oils or fats are used, wherein the principle chain lengths are C16 and higher. Preferred soap for use in the compositions of this invention has at least about 85 percent fatty acids having about 12 to 18 carbon atoms. The preferred soaps for use in the present invention should include at least about 30 percent saturated soaps, i.e., soaps derived from saturated fatty acids, preferably at least about 40 percent, more preferably about 50 percent, saturated soaps by weight of the fatty acid soap. Soaps can be classified into three broad categories which differ in the chain length of the hydrocarbon chain, i.e., the chain length of the fatty acid, and whether the fatty acid is saturated or unsaturated. For purposes of the present invention these classifications are: "Laurics" soaps which encompass soaps which are derived predominantly from C12 to C14 saturated fatty acid, i.e. lauric and myristic acid, but can contain minor amounts of soaps derived from shorter chain fatty acids, e.g., C10. Laurics soaps are generally derived in practice from the hydrolysis of nut oils such as coconut oil and palm kernel oil.

"Stearics" soaps which encompass soaps which are derived predominantly from C16 to C18 saturated fatty acid, i.e. palmitic and stearic acid but can contain minor level of saturated soaps derived from longer chain fatty acids, e.g., C20. Stearic soaps are generally derived in practice from triglyceride oils such as tallow, palm oil and palm stearin.

Oleic soaps which encompass soaps derived from unsaturated fatty acids including predominantly oleic acid, linoleic acid, myristoleic acid and palmitoleic acid as well as minor amounts of longer and shorter chain unsaturated and polyunsaturated fatty acids. Oleics soaps are generally derived in practice from the hydrolysis of various triglyceride oils and fats such as tallow, palm oil, sunflower seed oil and soybean oil. Coconut oil employed for the soap may be substituted in whole or in part by other "high-laurics" or "laurics rich" oils, that is, oils or fats wherein at least 45 percent of the total fatty acids are composed of lauric acid, myristic acid and mixtures thereof. These oils are generally exemplified by the tropical nut oils of the coconut oil class. For instance, they include: palm kernel oil, babassu oil, ouricuri oil, tucum oil, cohune nut oil, murumuru oil, jaboty kernel oil, khakan kernel oil, dika nut oil, and ucuhuba butter.

Disclosed composition comprsises 1 to 85 wt% of a fatty acid soap. Preferably the fatty acid soap is present in an amount not more than 80wt%, more preferably not more than 75wt%, still more preferably not more than 65wt%, further preferably not more than 55wt% and still further preferably not more than 45wt% and yet preferably not more than 35wt%.

The further preferred range of soap in the composition is 1 to 60% and most preferably 1 to 40% by weight of the composition.

The composition of the present invention preferably in the form of is in the form of a bar, liquid or gel.

The composition of the present invention is a synergistic antimicrobial composition. The synergy effects is observed by combining 0.1 to 100 ppm by weight of at least one silver compound, a salt of a sulphonic acid and 1 to 85% by weight of a salt of fatty acid. The synergistic antimicrobial composition of the present invention in the concentration range as mentioned above found to be effective against both gram-positive and gram-negative organisms.

Optional and preferred ingredients:
In addition to the ingredients described earlier, preferred embodiments of the cleansing compositions may also include other optional and preferred ingredients for their known benefits. The type and content will largely depend on the nature and type of cleansing composition as well as general principles of formulation science.
Where the composition is in the form of a bar of soap or a liquid soap, it is preferred that the composition contains free fatty acids. Preferred embodiments contain 0.01 wt% to 10 wt% free fatty acid, especially when major portion of the surfactant is soap based. Potentially suitable fatty acids are C8 to C22 fatty acids. Preferred fatty acids are C12 to C18, preferably predominantly saturated, straight-chain fatty acids. However, some unsaturated fatty acids can also be employed. Of course the free fatty acids can be mixtures of shorter chain length (e.g., C10 to C14) and longer chainlength (e.g., C16-C18) chain fatty acids. For example, one useful fatty acid is fatty acid derived from high-laurics triglycerides such as coconut oil, palm kernel oil, and babasu oil. The fatty acid can be incorporated directly or they can be generated in-situ by the addition of a protic acid to the soap during processing. Examples of suitable protic acids include: mineral acids such as hydrochloric acid and sulfuric acid, adipic acid, citric acid, glycolic acid, acetic acid, formic acid, fumaric acid, lactic acid, malic acid, maleic acid, succinic acid, tartaric acid and polyacrylic acid. However, care should be taken that the residual electrolyte in the bar does not substantially reduce the effectiveness of the anticracking agent. The level of fatty acid having a chain length of 14 carbon atoms and below should generally not exceed 5.0%, preferably not exceed about 1 % and most preferably be 0.8% or less based on the total weight of the continuous phase. Other optional compositions include one or more skin benefit agents. The term "skin benefit agent" is defined as a substance which softens or improves the elasticity, appearance, and youthfulness of the skin (stratum corneum) by either increasing its water content, adding, or replacing lipids and other skin nutrients; or both, and keeps it soft by retarding the decrease of its water content. Included among the suitable skin benefit agents are emollients, including, for example, hydrophobic emollients, hydrophilic emollients, or blends thereof. Water-soluble skin benefit agents may optionally be formulated into the liquid compositions of the invention. A variety of water-soluble skin benefit agents can be used and the level can be from 0 to 50% but preferably from 1 to 30% by weight of the composition. These materials include, but are not limited to, polyhydroxy alcohols. Preferred water soluble skin benefit agents are glycerin, sorbitol and polyethylene glycol.
Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflowerseed and cottonseed oils.
Water soluble/dispersible polymes is an optional ingredient that is highly preferred to be included in composition. These polymers can be cationic, anionic, amphoteric or nonionic types with molecular weights higher than 100,000 Dalton. They are known to increase the viscosity and stability of liquid cleanser compositions, to enhance in-use and after-use skin sensory feels, and to enhance lather creaminess and lather stability. Amount of the polymers, when present, may range from 0.1 to 10% by weight of the composition.

Examples of water soluble/or dispersible polymers include the carbohydrate gums such as cellulose gum, microcrystalline cellulose, cellulose gel, hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, xanthan gum and mixtures thereof; modified and nonmodified starch granules and pregelatinized cold water soluble starch; emulsion polymers such as Aculyn® 28, Aculyn® 22 or Carbopol® Aqua SF1; cationic polymer such as modified polysaccharides including cationic guar available from Rhone Poulenc under the trade name Jaguar@ C13S, Jaguar@ C14S, Jaguar@ C17, or Jaguar@ C16; cationic modified cellulose such as UCARE® Polymer JR 30 or JR 40 from Amerchol; N-Hance@ 3000, N-Hance® 3196, N-Hance® GPX 215 or N-Hance® GPX 196 from Hercules; synthetic cationic polymer such as Merquat® 100, Merquat® 280, Merquat® 281 and Merquat® 550 sold by Nalco; cationic starches such as StaLok® 100, 200, 300 and 400 sold by Staley Inc.; cationic galactomannans such as Galactasol® 800 series by Henkel, Inc.; Quadrosoft® LM-200; and Polyquaternium-24®. Also suitable are high molecular weight polyethylene glycols such as Polyox® WSR-205 (PEG 14M), Polyox® WSR-N-60K (PEG 45), and Polyox® WSR-301 (PEG 90M).

Preservatives can also be added into the compositions to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibility between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions may further include 0 to 10% by weight of opacifiers and pearlizers such as ethylene glycol distearate, titanium dioxide or Lytron® 621 (Styrene/Acrylate copolymer); all of which are useful in enhancing the appearance or properties of the product.

Soap bars in particular may contain particles that are greater than 50 µm in average diameter that help remove dry skin. Not being bound by theory, the degree of exfoliation depends on the size and morphology of the particles. Large and rough particles are usually very harsh and irritating. Very small particles may not serve as effective exfoliants. Such exfoliants used in the art include natural minerals such as silica, talc, calcite, pumice, tricalcium phosphate; seeds such as rice, apricot seeds, etc; crushed shells such as almond and walnut shells; oatmeal; polymers such as polyethylene and polypropylene beads, flower petals and leaves; microcrystalline wax beads; jojoba ester beads, and the like. These exfoliants come in a variety of particle sizes and morphology ranging from micron sized to a few mm. They also have a range of hardness. Some examples are talc, calcite, pumice, walnut shells, dolomite and polyethylene.

Advantageously, active agents other than skin conditioning agents defined above may be added to the composition. These active ingredients may be advantageously selected from bactericides, vitamins, anti-acne actives; anti-wrinkle, anti-skin atrophy and skin repair actives; skin barrier repair actives; non-steroidal cosmetic soothing actives; artificial tanning agents and accelerators; skin lightening actives; sunscreen actives; sebum stimulators; sebum inhibitors; anti-oxidants; protease inhibitors; skin tightening agents; anti-itch ingredients; hair growth inhibitors; 5-alpha reductase inhibitors; desquamating enzyme enhancers; anti-glycation agents; or mixtures thereof; and the like.

These active agents may be selected from water-soluble active agents, oil soluble active agents, pharmaceutically acceptable salts and mixtures thereof. The term "active agent" as used herein, means personal care actives which can be used to deliver a benefit to the skin and/or hair and which generally are not used to confer a skin conditioning benefit, such are delivered by emollients as defined above. The term "safe and effective amount" as used herein, means an amount of active agent high enough to modify the condition to be treated or to deliver the desired skin care benefit, but low enough to avoid serious side effects. The term "benefit," as used herein, means the therapeutic, prophylactic, and/or chronic benefits associated with treating a particular condition with one or more of the active agents described herein. What is a safe and effective amount of the active agent(s) will vary with the specific active agent, the ability of the active to penetrate through the skin, the age, health condition, and skin condition of the user, and other like factors.

A wide variety of active agent ingredients are useful for the inventive personal toilet bar compositions and include those selected from anti-acne actives, anti-wrinkle and anti-skin atrophy actives, skin barrier repair aids, cosmetic soothing aids, topical anesthetics, artificial tanning agents and accelerators, skin lightening actives, antimicrobial and antifungal actives, sunscreen actives, sebum stimulators, sebum inhibitors, anti-glycation actives and mixtures thereof and the like.

Anti-acne actives can be effective in treating acne vulgaris, a chronic disorder of the pilosebaceous follicles. Nonlimiting examples of useful anti-acne actives include the keratolytics such as salicylic acid (o-hydroxybenzoic acid), derivatives of salicylic acid such as 5-octanoyl salicylic acid and 4 methoxysalicylic acid, and resorcinol; retinoids such as retinoic acid and its derivatives (e.g., cis and trans); sulfur-containing D and L amino acids and their derivatives and salts, particularly their N-acetyl derivatives, mixtures thereof and the like.

Skin barrier repair actives are those skin care actives which can help repair and replenish the natural moisture barrier function of the epidermis. Non limiting examples of skin barrier repair actives include lipids such as cholesterol, ceramides, sucrose esters and pseudo-ceramides as described in European Patent Specification No. 556,957; ascorbic acid; biotin; biotin esters; phospholipids, mixtures thereof, and the like.

Artificial tanning actives can help in simulating a natural suntan by increasing melanin in the skin or by producing the appearance of increased melanin in the skin. Nonlimiting examples of artificial tanning agents and accelerators include dihydroxyacetaone; tyrosine; tyrosine esters such as ethyl tyrosinate and glucose tyrosinate; mixtures thereof, and the like.

Skin lightening actives can actually decrease the amount of melanin in the skin or provide such an effect by other mechanisms. Nonlimiting examples of skin lightening actives useful herein include aloe extract, alpha-glyceryl-L-ascorbic acid, aminotyrosine, ammonium lactate, glycolic acid, hydroquinone, 4 hydroxyanisole, mixtures thereof, and the like.

Also useful are sunscreen actives. Nonlimiting examples of sunscreens which are useful in the compositions of the present invention are those selected from the group consisting of octyl methoxyl cinnamate (Parsol MCX) and butyl methoxy benzoylmethane (Parsol 1789), 2-ethylhexyl p-methoxycinnamate, 2-ethylhexyl N,N-dimethyl-p-aminobenzoate, p-aminobenzoic acid, 2-phenylbenzimidazole-5sulfonic acid, oxybenzone, mixtures thereof, and the like.

Also useful are protease inhibitors. Protease inhibitors can be divided into two general classes: the proteinases and the peptidases. Proteinases act on specific interior peptide bonds of proteins and peptidases act on peptide bonds adjacent to a free amino or carboxyl group on the end of a protein and thus cleave the protein from the outside. The protease inhibitors suitable for use in the inventive personal toilet bar compositions include, but are not limited to, proteinases such as serine proteases, metalloproteases, cysteine proteases, and aspartyl protease, and peptidases, such as carboxypepidases, dipeptidases and aminopepidases, mixtures thereof and the like. Other useful active ingredients are skin tightening agents. Nonlimiting examples of skin tightening agents which are useful in the compositions of the present invention include monomers which can bind a polymer to the skin such as (meth) acrylic acid and a hydrophobic monomer comprised of long chain alkyl (meth) acrylates, mixtures thereof, and the like.

Active ingredients in the inventive personal toilet bar compositions may also include anti-itch ingredients. Suitable examples of anti-itch ingredients which are useful in the compositions of the present invention include hydrocortisone, methdilizine and trimeprazine, mixtures thereof, and the like.

Nonlimiting examples of hair growth inhibitors which are useful in the inventive personal toilet bar compositions include 17 beta estradiol, anti angiogenic steroids, curcuma extract, cycloxygenase inhibitors, evening primrose oil, linoleic acid and the like. Suitable 5-alpha reductase inhibitors such as ethynylestradiol and, genistine mixtures thereof, and the like.

Advantageously cationic skin feel agent(s) or polymer(s) are used from about 0.01, 0.1 or 0.2% by wt. to about 1, 1.5 or 2.0% by wt. in soap bars.

Cationic cellulose is available from Amerchol Corp. (Edison, N.J., USA) in their Polymer JR® and LR® series of polymers, as salts of hydroxyethyl cellulose reacted with trimethyl ammonium substituted epoxide, referred to in the industry (CTFA) as Polyquaternium® 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium® 24. These materials are available from Amerchol Corp. (Edison, N.J., USA) under the tradename Polymer LM-200®, and quaternary ammonium compounds such as alkyldimethylammonium halogenides.

A particularly suitable type of cationic polysaccharide polymer that can be used is a cationic guar gum derivative, such as guar hydroxypropyltrimonium chloride (Commercially available from Rhone-Poulenc in their JAGUAR® trademark series). Examples are JAGUAR® C13S, which has a low degree of substitution of the cationic groups and high viscosity, JAGUAR® C15, having a moderate degree of substitution and a low viscosity, JAGUAR® C17 (high degree of substitution, high viscosity), JAGUAR® C16, which is a hydroxypropylated cationic guar derivative containing a low level of substituent groups as well as cationic quaternary ammonium groups, and JAGUAR@ 162 which is a high transparency, medium viscosity guar having a low degree of substitution.

Particularly preferred cationic polymers are JAGUAR@ C13S, JAGUAR@ C15, JAGUAR@ C17 and JAGUAR@ C16 and JAGUAR@ C162, especially JAGUAR@ C13S. Other cationic skin feel agents known in the art may be used provided that they are compatible with the inventive formulation.

Other preferred cationic compounds that are useful in the present invention include amido quaternary ammonium compounds such as quaternary ammonium propionate and lactate salts, and quaternary ammonium hydrolyzates of silk or wheat protein, and the like. Many of these compounds can be obtained as the Mackine® Amido Functional Amines, Mackalene® Amido functional Tertiary Amine Salts, and Mackpro® cationic protein hydrolysates from the McIntyre Group Ltd. (University Park, III.).

In embodiments having a hydrolyzed protein conditioning agent, the average molecular weight of the hydrolyzed protein is preferably about 2500. Preferably 90% of the hydrolyzed protein is between a molecular weight of about 1500 to about 3500. In a preferred embodiment, MACKPRO® WWP (i.e. wheat germ amido dimethylamine hydrolyzed wheat protein) is added at a concentration of 0.1% (as is) in the bar.

The present invention also discloses a method of cleaning or disinfecting a surface comprising the steps of applying a composition of the present invention on to said surface and at least partially removing the composition from the surface. Preferably, the step of at least partially removing the composition is carried out less than 5 minutes after the step of applying the composition on the substrate.

The present invention also discloses a use of a composition of the present invention as disclosed above for improved antimicrobial benefit. The present invention further discloses a use of salt of a sulphonic acid in a composition comprising a silver compound and a salt of fatty acid to boost the antimicrobial action of the compostion, wherein the salt of a sulphonic acid is selected from sodium toluene sulphonates, sodium cumene sulphonates, sodium xylene sulphonates, naphthalene sulphonates or mixtures thereof.

The preferred use of the composition of the present invention is non-therapeutic and cosmetic.

The inventors have determined that the composition of the invention provides an antimicrobial action where the contact time of the antimicrobial actives with the surface is low, i.e. of the order of less than 5 minutes, preferably less than 2 minutes, further more preferably less than a minute and in many cases less than 15 seconds. Now the invention will be demonstrated by the following non limiting example.

The present invention now will be demonstrated by way of following non-limiting examples.

### Examples:

### Invitro experiments with the ingredients of the composition of the present invention to find out the antimicrobial efficacy:

The following protocol was used to evaluate biocidal activity.
IN-VITRO TIME-KILL PROTOCOL - ASTM 2783

Experiments were carried out with the individual ingredients of the composition and their combinations in a neat system.

The following samples were prepared for these experiments:

### Example A (Comparative Example)

As control, 1.5 g of sodium laurate was dissolved in 100 mL of demineralized water. To this 0.1 mg (i.e. 1 ppm) of Ag₂O (as silver DTPA complex) was added. 10 mL of this solution was used for antimicrobial efficacy testing.

### Example B (Comparative Example)

In this example, 1.5 g of sodium laurate and 1.5 g of sodium alpha olefin sulphonate (obtained from Godrej Chemicals) was dissolved in 100 mL of demineralized water. To this 0.1 mg (i.e. 1 ppm) of Ag2O (as silver DTPA complex) was added. 10 mL of this solution was used for antimicrobial efficacy testing.

### Example 1

In this example, 1.5 g of sodium laurate and 1.5 g of sodium naphthalene sulphonate (Aldrich) was dissolved in 100 mL of demineralized water. To this 0.1 mg (i.e. 1 ppm) of Ag2O (as silver DTPA complex) was added. 10 mL of this solution was used for antimicrobial efficacy testing.

### Example 2

This is same as Example 1, only difference is that the use of Sodium Toluene sulphonate (Aldrich; Cat No: 15252-6) as a salt of a sulphonic acid.

The silver DTPA complex as mentioned above was prepared by using 1.500 g of Silver oxide powder with 22.5g of 40% Na₅DTPA (Sodium salt of diethylene triamine pentaacetic acid). The above mixture was stirred and heated at ∼ 45°C in a water bath for 10 minutes. Any particulates observed are broken with glass rod. After that 975g of water was added water stirring ambient temp (∼25°C). The stirring was continued for 10 minutes. After that 0.8g of powdered lauric acid was added and stirred for 30 minutes. The resulting mixture was centrifuged to separate out the supernatant from the residue for 5 minutes. The supernatant is silver DTPA complex used in the experiments.

Preparation of the bacterial culture:
S. aureus ATCC 6538 was used in the study which is a gram positive bacteria. The bacteria were grown overnight on Tryptic soya agar (TSA) plate. The bacterial cell density was then adjusted at 620 nm to a pre-calibrated optical density to get the final count of 10⁹ cfu/mL in saline (0.86% NaCI) by using a spectrophotometer.

Assay Protocol:
9.9 mL of the composition of different examples (as stated above) was taken in different sample containers to each of those container 0.1mL of bacterial culture was added just before performing the assay and mixed well to obtain a mixture. A timer was started immediately after the addition of the culture. The mixture was kept for a specific contact of 10 seconds and 30 seconds.

At the end of the each contact time (10 seconds and 30 seconds), the antibacterial activity of the samples was neutralized immediately, by addition of 1 mL each of the above mixture to 9 mL of an appropriate neutralizing broth which is validated for the test system. The neutralized samples were then serially diluted upto 5 dilution in neutralizer broth and plated on TSA (40gpL - Difco) in duplicates.

The log reduction was calculated by comparing with the bacterial control. The bacterial control used for this purpose was a mixture prepared by addition of 0.1 mL of bacterial culture to 9.9 mL of saline; the mixture was then serially diluted and plated on TSA. After solidification of the TSA plates, the plates were incubated at 37°C for 48 hours. The colonies on the plates were counted.

The results are summarized below in Table 1:

**Table 1:**

| Example | Log₁₀ reduction | |
|---|---|---|
| | 10 seconds (contact time) | 30 seconds (contact time) |
| A | 1.85 ± 0.02 | 3.42± 0.20 |
| B | 2.11 ± 0.07 | 3.79 ± 0.40 |
| 1 | 2.82 ± 0.19 | 4.52 ± 0.41 |
| 2 | 3.39 ± 0.08 | 5.15 ± 0.14 |
| | | |

From the above table, it is evident that the composition with silver, a salt of an aromatic sulphonic acid and a fatty acid as per the present invention (Example 1 and 2) provides much higher Log reduction than the composition that comprises only silver and a salt of fatty acid (example A) and a combination of a salt of an aliphatic sulphonic acid with a fatty acid (Example B). The effect is more pronounced at short contact time of 10 seconds.

It is now therefore clear from the above description and the examples that it is now possible by way of present invention to provide an antimicrobial cleansing composition that provides biocidal activity in relatively short contact times of 1 minute to 10 seconds at very low concentration of silver compound.

## Claims

1. An antimicrobial composition comprising:
a) 0.1 to 100 ppm by weight of a silver compound;
b) a salt of a sulphonic acid ; and,
c) 1 to 85% by weight of a salt of fatty acid;
wherein, the salt of a sulphonic acid is selected from sodium toluene sulphonates, sodium cumene sulphonates, sodium xylene sulphonates, naphthalene sulphonates or mixtures thereof.

2. A composition as claimed in claim 1 wherein the amount of the salt of a sulphonic acid is in the range of 0.1 to 20% by weight of the composition.

3. A composition as claimed in any one of the preceding claims wherein the silver compound is selected from group consisting of silver oxide, silver nitrate, silver acetate, silver sulfate, silver benzoate, silver salicylate, silver carbonate, silver citrate, silver phosphate.

4. A composition as claimed in any one of the preceding claims 1 to 2 wherein the silver compound is a complex of silver.

5. A composition as claimed in claim 4 wherein the complex of silver is formed by reacting silver with one or more of a chelating agent.

6. A composition as claimed in claim 5 wherein said chelating agent is selected from ethylene diamine tetraacetic acid (EDTA), ethylene diamine dissuccinate (EDDS), N,N-bis(carboxymethyl) glutamic acid (GLDA), Diethylenetriaminepentaacetic acid (DTPA), Nitrilotriacetic acid (NTA) or Ethanoldiglycinic acid ((EDG).

7. A composition as claimed in any one of the preceding claims 5 or 6 wherein the molar ratio of silver to said chelating agent is 1:0.25 to 1:10.

8. A composition as claimed in any one of the preceding claims further comprising a cosmetically acceptable base.

9. A composition as claimed in any one of the preceding claims in the form of a bar, liquid or gel.

10. A non-therapeutic method of cleaning or disinfecting a surface comprising the steps of applying a composition as claimed in any one of the preceding claims on to said surface and at least partially removing the composition from the surface.

11. A non-therapeutic method as claimed in claim 10 wherein the step of at least partially removing the composition is carried out less than 5 minutes after the step of applying the composition on the substrate.

12. The non-therapeutic use of a composition according to any one of claims 1 to 9 for improved antimicrobial benefit.

13. The non-therapeutic use of salt of a sulphonic acid in a composition comprising 0.1 to 100 ppm by weight of a silver compound and 1 to 85% by weight of a salt of fatty acid to boost the antimicrobial action of the composition; wherein the salt of a sulphonic acid is selected from sodium toluene sulphonates, sodium cumene sulphonates, sodium xylene sulphonates, naphthalene sulphonates or mixtures thereof.

## Patentansprüche

1. Antimikrobielle Zusammensetzung, umfassend:
a) 0,1 bis 100 Gewichts-ppm einer Silberverbindung;
b) ein Salz einer Sulfonsäure; und
c) 1 bis 85 Gewichts-% eines Salzes von Fettsäure;
wobei das Salz einer Sulfonsäure ausgewählt ist aus Natriumtoluolsulfonaten, Natriumcumolsulfonaten, Natriumxylolsulfonaten, Naphthalinsulfonaten oder Mischungen davon.

2. Zusammensetzung wie in Anspruch 1 beansprucht, wobei die Menge des Salzes einer Sulfonsäure im Bereich von 0,1 bis 20 Gewichts-% der Zusammensetzung liegt.

3. Zusammensetzung wie in irgendeinem der vorhergehenden Ansprüche beansprucht, wobei die Silberverbindung ausgewählt ist aus einer Gruppe bestehend aus Silberoxid, Silbernitrat, Silberacetat, Silbersulfat, Silberbenzoat, Silbersalicylat, Silbercarbonat, Silbercitrat, Silberphosphat.

4. Zusammensetzung wie in irgendeinem der vorhergehenden Ansprüche 1 bis 2 beansprucht, wobei die Silberverbindung ein Silberkomplex ist.

5. Zusammensetzung wie in Anspruch 4 beansprucht, wobei der Silberkomplex durch Umsetzung von Silber mit einem oder mehreren Chelatbildnern gebildet wird.

6. Zusammensetzung wie in Anspruch 5 beansprucht, wobei der Chelatbildner ausgewählt ist aus Ethylendiamintetraessigsäure (EDTA), Ethylendiamindisuccinat (EDDS), N,N-Bis(carboxymethyl)glutaminsäure (GLDA), Diethylentriaminpentaessigsäure (DTPA), Nitrilotriessigsäure (NTA) oder Ethanoldiglycinsäure (EDG).

7. Zusammensetzung wie in irgendeinem der vorhergehenden Ansprüche 5 oder 6 beansprucht, wobei das Molverhältnis von Silber zu dem Chelatbildner 1:0,25 bis 1:10 beträgt.

8. Zusammensetzung wie in irgendeinem der vorhergehenden Ansprüche beansprucht, ferner umfassend eine kosmetisch akzeptable Basis.

9. Zusammensetzung wie in irgendeinem der vorhergehenden Ansprüche beansprucht in Form eines Riegels, einer Flüssigkeit oder eines Gels.

10. Nichttherapeutisches Verfahren zum Reinigen oder Desinfizieren einer Oberfläche, umfassend die Schritte des Aufbringens einer Zusammensetzung wie in irgendeinem der vorhergehenden Ansprüche beansprucht auf die Oberfläche und des zumindest teilweisen Entfernens der Zusammensetzung von der Oberfläche.

11. Nichttherapeutisches Verfahren wie in Anspruch 10 beansprucht, wobei der Schritt des zumindest teilweisen Entfernens der Zusammensetzung weniger als 5 Minuten nach dem Schritt des Aufbringens der Zusammensetzung auf das Substrat durchgeführt wird.

12. Nichttherapeutische Verwendung einer Zusammensetzung nach irgendeinem der Ansprüche 1 bis 9 für einen verbesserten antimikrobiellen Nutzen.

13. Nichttherapeutische Verwendung von Salz einer Sulfonsäure in einer Zusammensetzung, umfassend 0,1 bis 100 Gewichts-ppm einer Silberverbindung und 1 bis 85 Gewichts-% eines Salzes von Fettsäure, um die antimikrobielle Wirkung der Zusammensetzung zu verstärken, wobei das Salz einer Sulfonsäure ausgewählt ist aus Natriumtoluolsulfonaten, Natriumcumolsulfonaten, Natriumxylolsulfonaten, Naphthalinsulfonaten oder Mischungen davon.

## Revendications

1. Composition antimicrobienne comprenant :
a) 0,1 à 100 ppm en masse d'un composé d'argent ;
b) un sel d'un acide sulfonique ; et,
c) 1 à 85 % en masse d'un sel d'acide gras ;
dans laquelle, le sel d'un acide sulfonique est choisi parmi des toluènesulfonates de sodium, cumènesulfonates de sodium, xylènesulfonates de sodium, sulfonates de naphtalène ou mélanges de ceux-ci.

2. Composition selon la revendication 1, dans laquelle la quantité du sel d'un acide sulfonique se trouve dans un intervalle de 0,1 à 20 % en masse de la composition.

3. Composition selon l'une quelconque des revendications précédentes, dans laquelle le composé d'argent est choisi dans le groupe consistant en oxyde d'argent, nitrate d'argent, acétate d'argent, sulfate d'argent, benzoate d'argent, salicylate d'argent, carbonate d'argent, citrate d'argent, phosphate d'argent.

4. Composition selon l'une quelconque des revendications 1 à 2 précédentes, dans laquelle le composé d'argent est un complexe d'argent.

5. Composition selon la revendication 4, dans laquelle le complexe d'argent est formé par réaction d'argent avec un ou plusieurs d'un agent chélatant.

6. Composition selon la revendication 5, dans laquelle ledit agent chélatant est choisi parmi l'acide éthylènediamine tétraacétique (EDTA), disuccinate d'éthylènediamine (EDDS), acide N,N-bis(carboxyméthyl)glutamique (GLDA), acide diéthylènetriamine-pentaacétique (DTPA), acide nitrilotriacétique (NTA) ou acide éthanol-diglycinique (EDG).

7. Composition selon l'une quelconque des revendications 5 ou 6 précédentes, dans laquelle le rapport molaire d'argent audit agent chélatant est de 1:0,25 à 1:10.

8. Composition selon l'une quelconque des revendications précédentes comprenant de plus une base cosmétiquement acceptable.

9. Composition selon l'une quelconque des revendications précédentes dans la forme d'une barre, d'un liquide ou d'un gel.

10. Procédé non-thérapeutique de nettoyage ou désinfection d'une surface comprenant les étapes d'application d'une composition selon l'une quelconque des revendications précédentes sur ladite surface et d'élimination au moins partielle de la composition de la surface.

11. Procédé non-thérapeutique selon la revendication 10, dans lequel l'étape d'élimination au moins partielle de la composition est réalisée moins de 5 minutes après l'étape d'application de la composition sur le substrat.

12. Utilisation non-thérapeutique d'une composition selon l'une quelconque des revendications 1 à 9 pour un bénéfice antimicrobien amélioré.

13. Utilisation non-thérapeutique de sel d'un acide sulfonique dans une composition comprenant 0,1 à 100 ppm en masse d'un composé d'argent et 1 à 85 % en masse d'un sel d'acide gras pour intensifier l'action antimicrobienne de la composition ; dans laquelle le sel d'un acide sulfonique est choisi parmi des toluènesulfonates de sodium, cumènesulfonates de sodium, xylènesulfonates de sodium, sulfonates de naphtalène ou mélanges de ceux-ci.
